# EUROPEAN PATENT SPECIFICATION

(11) **EP 2 547 358 B1**
(45) Date of publication and mention of the grant of the patent: **02.11.2016**
(21) Application number: 11714559.9
(22) Date of filing: 15.04.2011
(51) Int. Cl.: A61K 39/00, A61K 39/29

(54) **METHOD OF VACCINATION**
Impfverfahren
Procédé de vaccination

(30) Priority: 16.04.2010 EP 10305399
(43) Date of publication of application: 23.01.2013
(73) Proprietor: DBV Technologies, 92120 Montrouge (FR)
(72) Inventor: SIEGRIST, Claire-Anne, CH-14 Geneva (CH); MONDOULET, Lucie, F-92320 Chatillon (FR)
(74) Representative: Becker, Philippe
(86) International application number: PCT/EP2011/055991
(87) International publication number: WO 2011/128430

(56) References cited:
- WO-A1-2009/080933
- WO-A2-2004/030696
- WO-A2-2007/122226
- MISHRA V ET AL: "Development of novel fusogenic vesosomes for transcutaneous immunization", VACCINE, ELSEVIER LTD, GB LNKD- DOI:10.1016/J.VACCINE.2006.04.030, vol. 24, no. 27-28, 7 July 2006 (2006-07-07), pages 5559-5570, XP025151744, ISSN: 0264-410X [retrieved on 2006-07-07] cited in the application
- HAMMOND S A ET AL: "Transcutaneous immunization: T cell responses and boosting of existing immunity", VACCINE, ELSEVIER LTD, GB LNKD- DOI:10.1016/S0264-410X(00)00506-5, vol. 19, no. 17-19, 21 March 2001 (2001-03-21), pages 2701-2707, XP004231100, ISSN: 0264-410X cited in the application
- DIOSZEGHY V ET AL: "T.118. Repeated Applications of Peanut Protein Extracts During Epicutaneous Immunotherapy Induce a Change of Cytokine Response from Th2 to Mixed TH2/TH1 in Sensitized Mice", CLINICAL IMMUNOLOGY, ACADEMIC PRESS, US LNKD- DOI:10.1016/J.CLIM.2009.03.251, vol. 131, 1 January 2009 (2009-01-01), page S86, XP026084644, ISSN: 1521-6616 [retrieved on 2009-01-01]
- COPE R B ET AL: "Effect on systemic antibody concentrations of topical application of choleratoxin to skin of sheep", AUSTRALIAN VETERINARY JOURNAL, AUSTRALIAN VETERINARY ASSOCIATION, BRUNSWICK, AU LNKD- DOI:10.1111/J.1751-0813.2000.TB10540.X, vol. 78, no. 2, 1 February 2000 (2000-02-01), pages 121-123, XP008126584, ISSN: 0005-0423 [retrieved on 2008-03-10]
- ZHANG JIANFENG ET AL: "Topical application of Escherichia coli particles over-producing pathogen-derived antigens as a simple vaccination modality in compliance with evolutionary medicine", DRUG TARGETS - INFECTIOUS DISORDERS, BENTHAM SCIENCE PUBLISHERS LTD, NL, vol. 8, no. 3, 1 September 2008 (2008-09-01), pages 189-194, XP008126581, ISSN: 1871-5265
- DIOSZEGHY V ET AL: "A New Delivery System for Epicutaneous Immunotherapy Promoting Specific Antigen Uptake by Langerhans Cells", CLINICAL IMMUNOLOGY, ACADEMIC PRESS, US, vol. 135, 1 January 2010 (2010-01-01), page S93, XP027049078, ISSN: 1521-6616 [retrieved on 2010-01-01]

## Description

### FIELD OF THE INVENTION

The present invention relates to improved methods for vaccination of a subject. Particularly, the present invention discloses the use of skin antigen application to amplify and improve a pre-existing immunity against a selected pathogen in a subject. The present invention discloses the use of skin application in combination with conventional vaccination or priming for improved immunization or vaccination of a subject against a selected pathogen.

### BACKGROUND OF THE INVENTION

Vaccination of a subject is a method of generating a protective immune response against a selected pathogen. Vaccination may be preventive, i.e., conducted prior to a subject's exposure to the pathogen, and/or curative, i.e., conducted after exposure, in order to increase, expand or stimulate the subject's immune defense against the pathogenic agent.

Conventional vaccination comprises the parenteral, nasal or oral administration of an antigen to a subject. Most vaccination programs comprise the (repeated) parenteral administration (e.g. injection) to a subject of a selected antigen specific of the pathogen, thereby inducing or amplifying the subject's immune system against the pathogen. Conventional vaccination may also be oral or nasal.

Vaccination generally comprises a repeated administration protocol, including a priming administration followed by one or several boost administrations. In most cases, such a prime-boost vaccination, requiring more than one time immunization, is performed using the same vaccines multiple times as homologous boosts. Also, in conventional vaccination programs, the antigen is generally used in combination with one or more adjuvants, in order to generate the appropriate immune response.

Wu et al., (2005) describes a conventional heterologous prime-boost method wherein the priming is performed by injection of a first antigen (a plasmid DNA encoding an antigen) and boosting is performed by injection of a second preparation (a replication-incompetent adenovirus vector encoding the same antigen). This approach has been tested in the development of vaccines against a number of viral pathogens including HIV, hepatitis C virus, Ebola virus, and Venezuelan equine encephalitis virus. DNA priming followed by adenovirus boosting has also been evaluated as an approach for immunizing against a bacterial disease (McConnell et al., 2007).

Conventional vaccination has been investigated using different types of antigens, such as e.g., proteins, peptides, cells, inactivated pathogens, toxoids, virus-like particles, etc.

Topical application of vaccine formulations on the skin has also been proposed.

In particular, Partidos et al. 2003, have shown that an immune response may be caused by epicutaneous route, subject to specific administration conditions, the use of a particular adjuvant and a pre-treatment of the skin.

US 7,378,097 also relates to vaccination by application on pre-treated skin of adjuvanted antigen compositions.

Mishra et al, 2006, propose a system of transcutaneous immunization using novel vesicular constructs, fusogenic vesosomes, which deliver antigens via topical administration. However this vesicular system is a liquid, non-occlusive system, and needs applying antigen together with an adjuvant onto hydrated skin.

Hammond et al, 2001, propose a method oftranscutaneous vaccination of mice with cholera toxin and adjuvant, by which liquid solutions of adjuvanted antigens are applied topically to hydrated skin.

All the above methods require the use of an adjuvant to cause an immune response. Also, they use liquid preparations, and the nature of the immune responses provoked are neither controlled nor analyzed.

WO2007/122226 and WO2009/080933 disclose epicutaneous immunization using antigen preparations devoid of adjuvant applied on intact skin. This application shows that a protective response may be obtained without added adjuvant and without skin pre-treatment, through skin application of an antigen.

Despite the above strategies and positive responses for vaccinating or protecting mammalian subjects, there is still a need in the art for improved immunization methods, which are more convenient for the patient and induce a suitable and efficient immune response to prevent or treat diseases such as infectious diseases.

### SUMMARY OF THE INVENTION

The present application provides a novel method of developing immunity against pathogens in mammalian subjects. More particularly, the invention provides methods of amplifying and/or orienting pre-existing immunity in subjects using epicutaneous antigen application. As will be disclosed in the present application, this method leads to a strong TH1-oriented immune response and is much more convenient than current vaccination protocols. In particular, the invention shows that a cutaneous boost causes both an amplification of a pre-existing immune response and a TH1 polarization of the immune response, which is particularly suited for efficient vaccination. The invention also discloses a method for stimulating and/or TH1-orienting a pre-existing immune response against a pathogen in a mammalian subject, the method comprising applying on the skin of a subject having a pre-existing immune response against a pathogen, an antigen specific for the pathogen, said skin application leading to a stimulation and TH1-orientation of the response.

In particular, the invention relates to a preparation of an antigen as defined in the claims. The invention also relates to a preparation of an antigen specific for a pathogen for use in amplifying and/or Th1 orienting a pre-existing immune response against said pathogen in a mammalian subject by application of said preparation to the skin of the subject. Preferably the antigen preparation is dry and/or non-adjuvanted.

Preferably, the antigen preparation is applied on intact skin of the subject. Also, in a preferred embodiment, the antigen preparation is applied on the skin of the subject using an occlusive patch device.

The invention also relates to the use of a preparation of an antigen specific for a pathogen for the manufacture of a medicament for amplifying and preferably Th1-orienting a pre-existing immune response against said pathogen in a mammalian subject by application of said preparation to the skin of the subject. Preferably the antigen is dry and/or non-adjuvanted.

As will be further disclosed in the present application, the pre-existing immunity may result from the prior conventional vaccination of the subject against said pathogen, from the prior conventional immune priming of the subject against said pathogen, and/or from the prior natural exposure of the subject to said pathogen.

The present invention also discloses a method of vaccinating a mammalian against a selected pathogen, the method comprising (i) the conventional administration to the mammalian of an antigen, specific for the pathogen, to cause or stimulate an immune response to said pathogen, and (ii) the subsequent application(s), on the skin of the mammalian, of an antigen specific for the pathogen. As will be documented, such skin application allows amplification and Th1 orientation of the immune response. Step (ii) may be performed either shortly after step (i), or later, as long as an immunity against the pathogen still exists in the subject. Step (ii) typically consists in 1, 2 or 3 boost treatments, which may be performed at various time intervals from each other, depending e.g., on the pathogenic agent, typically between 1-18 months.

A further object of the present invention thus relates to an antigen preparation for use in vaccinating a mammalian against a selected pathogen, the antigen being first conventionally administered to the mammalian to cause or stimulate an immune response to said pathogen, and subsequently applied on the skin of the mammalian.

This invention also discloses a method of boosting an immune response in a mammalian subject immunized against a pathogen, the method comprising applying on the skin of said subject an antigen specific for the pathogen, preferably in a dry non adjuvanted preparation. More preferably, the antigen preparation is non adjuvanted and applied on intact skin area of the subject.

This invention also discloses a method of amplifying and Th1-orienting an immune response against a pathogen in a pre-immunized mammalian subject, the method comprising applying on intact skin of said pre-immunized subject an antigen specific for the pathogen, preferably in a dry and/or non adjuvanted preparation.

The invention also relates to an antigen specific for a pathogen (preferably in a dry and/or non adjuvanted preparation) for use in amplifying and Th1-orienting an immune response against said pathogen in a pre-immunized mammalian subject by application(s) of the antigen to the skin of the subject.

The invention also discloses a method for inducing or stimulating a pathogen-specific, Th1-oriented, immune response in a mammalian subject, the method comprising the conventional administration to the mammalian of an antigen specific for the pathogen to cause or stimulate an immune response against said pathogen; and subsequently the application(s), on intact skin of the mammalian, of an antigen specific for the pathogen, allowing amplification of a Th1-oriented immune response.

The invention also discloses a method of amplifying a pre-existing immune response against a pathogen in a mammalian subject, the method comprising applying a dry and non adjuvanted preparation of an antigen specific for said pathogen to intact skin of the subject.

The invention also discloses a method of boosting an immune response in a mammalian subject pre-immunized against a pathogen, the method comprising applying an antigen preparation specific for said pathogen on the skin of said subject under conditions allowing amplification and Th1 orientation of the immune response.

This invention also discloses a composition comprising an injectable preparation of an antigen specific for a selected pathogen; and a dry, non-adjuvanted preparation of an antigen specific for said selected pathogen, suitable for administration to the skin, for separate, sequential application to a mammalian subject.

The invention also discloses a kit comprising an injectable preparation of an antigen specific for a selected pathogen; and a dry, non-adjuvanted preparation of an antigen specific for said selected pathogen, suitable for administration to the skin.

The invention may be used in any mammalian, particularly any human subject, to induce or amplify a protective and/or curative immune response against any pathogen. The invention may also be used in the veterinary field, to treat animals such as cattle or pets, including cows, pigs, horses, dogs, cats, etc. It is particularly suited to vaccinate against infectious pathogens.

### LEGEND TO THE FIGURES

Figure 1: Influence of skin boost on anti-HBsAg IgG1 responses
Figure 2: Influence of skin boost on anti-HBsAg IgG2a responses
Figure 3: Cytokine-producing-cells in mice boosted twice by skin application or injection
Figure 4: Cytokine secretion in mice boosted twice by skin application or injection

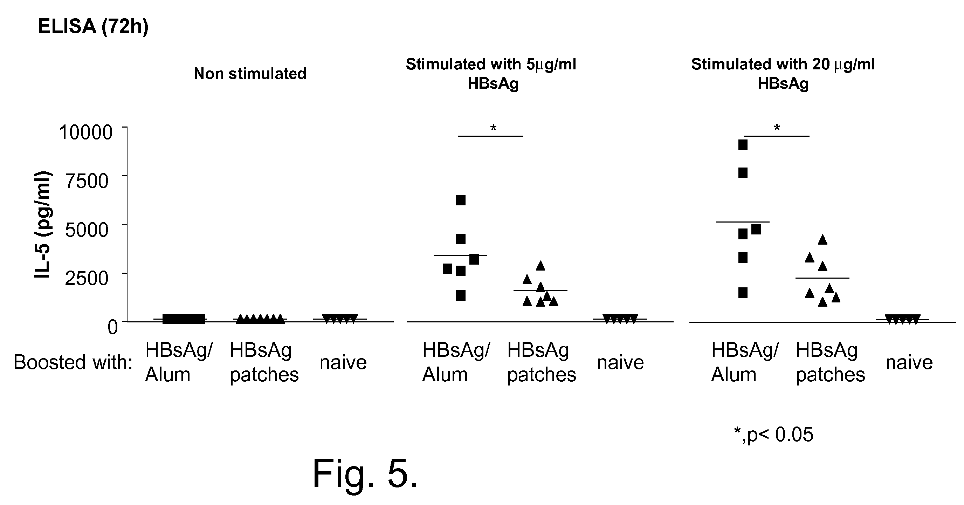

Figure 5: IL-5 responses in mice boosted twice by skin application or injection (ELISA)

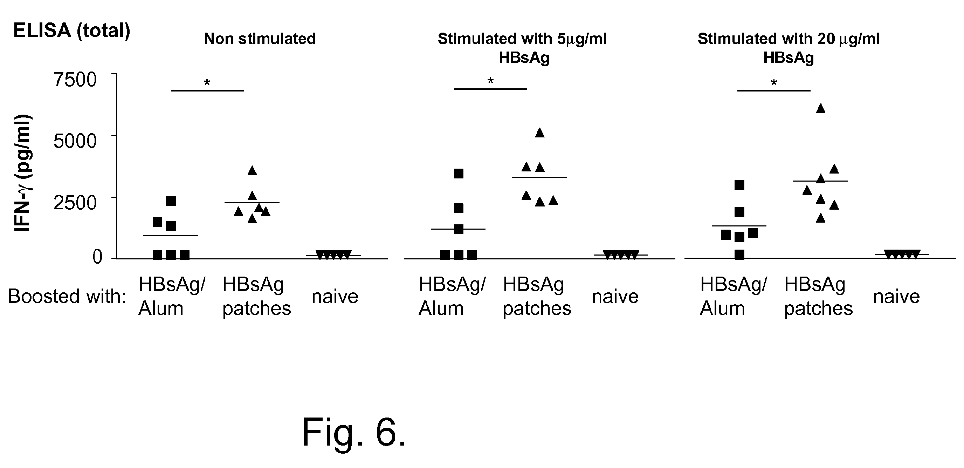

Figure 6: IFN-γ responses in mice boosted twice by skin application or injection (ELISA)
Figure 7: Comparison of the immunological response induced on intact and tape-stripped skin: level of specific IgE (7A) and IgG2a (7B)
Figure 8: Comparison of the immunological response induced on intact and tape-stripped skin: eosinophil counts in esophagus
Figure 9: Comparison of the immunological response induced on intact and tape-stripped skin: mRNA Expression of cytokines in esophagus: eotaxin (Fig. 9A), IL-5 (Fig. 9B), and IL-13 (Fig. 9C)
Figure 10: Comparison of the immunological response induced on intact and tape-stripped skin: mRNA Expression in Esophagus : GATA-3 (Fig. 10A), Foxp3 (Fig. 10B)
Figure 11: Comparison of the immunological response induced on intact and tape-stripped skin: histological data - villosity/crypt ratio in jejunum

### DETAILED DESCRIPTION OF THE INVENTION

The present invention relates to compositions and methods for improving vaccination. Particularly, the invention relates to the stimulation of a pre-existing immunity in a subject using epicutaneous antigen delivery. The invention discloses improved "prime-boost" vaccination methods in which the immune system is induced by conventional administration of a priming antigen composition, and boosted by epicutaneous application of a boosting antigen composition. The present invention also relates to methods for producing a protective TH1-oriented immune response against a pathogen in a pre-immunised subject using epicutaneous antigen administration(s). This method is safe for the patient, practical, and produces a strong Th1-oriented immune response in mammalian subjects.

The present invention shows that a specific immune reaction provoked by conventional priming, prior vaccination and/or natural exposure to a pathogen, which is TH2-oriented, can be efficiently amplified and TH1-polarized upon epicutaneous boosting of the subject. The results presented show that an epicutaneous boosting with no adjuvant and no skin pre-treatment leads to an effective amplification and TH1-orientation of a pre-existing immune response. The results further show that such a response is particularly pronounced when the boost is performed using a dry antigen preparation and an occlusive skin device.

The invention may be used to induce or enhance an immune response and to produce immunity to any pathogen. The invention can also be used to prevent or treat diseases provoked by a pathogen.

The present disclosure will be best understood by reference to the following definitions:

### Definitions

As used therein, "conventional" administration or vaccination designates the parenteral, oral or nasal administration or vaccination. Parenteral administration can be performed by injection (e.g., intramuscular, intradermal, intravenous or subcutaneous), puncture, and/or transdermal administration. A preferred parenteral administration route is through injection.

Epicutaneous or skin application designates the application of the antigen on the skin of the subject under conditions allowing a contact with the surface of the skin. Skin application according to the present invention is preferably performed on intact (or non pre-treated) skin. Skin application should be maintained for a period of time sufficient to allow penetration of an antigen in the superficial layer(s) of the skin and/or contact of the antigen with immune cells.

Within the context of the present invention, the term "intact skin" indicates that the integrity of the stratum corneum layer should be substantially maintained. Previous work has suggested that, for efficient epicutaneous immunotherapy and enhanced antigen penetration and activation of the immune response, it is necessary to remove the stratum corneum of the skin (e.g., through abrasives or deep tape-stripping), or to pass through the stratum corneum (e.g., using microneedles) (Frerichs et al., Vaccine 2008, p2782 ; Strid et al, Eur J. Immunol 2004 p2100). These previous work indicate that, by altering the integrity of the stratum corneum, keratinocytes and Langerhans cells are activated or stimulated, leading to a better response of the organism. In contrast with these prior observations, the invention shows that it is preferable to apply antigens on intact (i.e., non-pre-treated) skin, e.g., on a surface or portion of the skin where the integrity of the stratum corneum is essentially maintained. The invention indeed shows that, to avoid a biased Th2 immune response, it is important to maintain the integrity of the stratum corneum and natural activation state of keratinocytes and Langerhans cells which are located below the stratum corneum. By maintaining this integrity, the response obtained is highly oriented in the sense of tolerance. Accordingly, although gentle cleaning of the skin surface may be performed at the site of application, e.g., hydration, water cleaning, or very gentle single stripping, to remove e.g., corneocytes, the skin should not be pre-treated so as to maintain substantial integrity of the stratum corneum. In particular, tape-stripping or strong abrasion of the skin should not be performed, since such pre-treatments disrupt, or remove all or part of the stratum corneum and cause stimulation of keratinocytes. Similarly, perforation of the stratum corneum should be avoided.

As described herein, the term "vaccinating" designates typically the sequential administration of one or more antigens to a mammal, to produce and/or enhance an immune response against the antigen(s). The sequential administration includes a priming immunization followed by one or several boosting immunizations.

Within the context of the present invention, the term "pathogen" refers to any agent that can cause a pathological condition. Examples of "pathogens" include, without limitation, cells (e.g., bacteria cells, diseased mammal cells, cancer mammal cells), fungus, parasites, viruses, prions or toxins. Preferred pathogens are infectious pathogens. In a particular embodiment, the infectious pathogen is a virus, such as hepatitis virus, rotavirus, chicken pox virus, influenza, cytomegalovirus, flu, HIV, Ebola virus, or Rabies.

Specific examples of infectious pathogens include, without limitation, C. diphteriae, C. tetani, B. Pertussis, Poliovirus, Mumps, Rubella, Varicella (Chicken pox), Streptococcus pneumoniae, Rotavirus, HPV (Human Papillomavirus), African trypanosomiasis (sleeping sickness), Anthrax, Avian influenza (" bird flu"), Buruli ulcer disease, Cholera, Crimean-Congo haemorrhagic fever, Dengue and dengue haemorrhagic fever, Ebola haemorrhagic fever, Enteroviruses - non polio, Haemophilus influenzae type B (HiB), Hendra virus, Hepatitis A, Hepatitis B, Hepatitis C, Hepatitis D, Hepatitis E, Influenza (Seasonal), Lassa fever, Legionellosis, Leprosy, Malaria, Marburg haemorrhagic fever, Measles, Meningococcal meningitis, Nipah virus, Plague, Poliomyelitis, Rift Valley fever, Smallpox, Tuberculosis or Yellow fever pathogen.

An antigen, as used therein, designates any molecule which can cause a T-cell or B-cell immune response in a subject. An antigen specific for a pathogen is, typically, an element obtained or derived from said pathogen, which contains an epitope, and which can cause an immune response against the pathogen. Depending on the pathogenic agent, the antigen may be of various nature, such as a (poly)peptide, protein, nucleic acid, lipid, cell, etc. Live weakened forms of pathogens (e.g., bacteria, viruses), or killed or inactivated forms thereof may be used as well, or purified material therefrom such as proteins, peptides, lipids, etc. The antigen may be naturally-occurring or artificially created. It may be exogenous to the treated mammal, or endogenous (e.g., tumor antigens). The antigen may be produced by techniques known *per se* in the art, such as for instance synthetic or recombinant technologies, or enzymatic approaches.

Specific examples of antigens suitable for use in the present invention include, e.g., viral antigens, preferably viral surface antigens, such as e.g., Hepatitis B or A Virus antigen (HBsAg, HAsAg). Other antigens include tumour-associated antigens, i.e., antigen expressed by a tumour cell but not by normal cells, bacterial proteins, etc.

In a particular embodiment, the antigen is a protein, polypeptide and/or peptide. The terms "polypeptide", "peptide" and "protein" are used interchangeably herein to refer to a polymer of amino acid residues. The terms also apply to amino acid polymers in which one or more amino acid residues may be modified or non-naturally occurring residues, such as an artificial chemical mimetic of a corresponding naturally occurring amino acid. It should be understood that the term "protein" also includes fragments or variants of different antigens, such as epitope-containing fragments, or proteins obtained from a pathogen and subsequently enzymatically, chemically, mechanically or thermally modified.

As will be discussed further below, the antigen may be in various states, such as liquid or dry. Typically, for skin application(s), the antigen is in dry state, while for conventional vaccination or priming, the antigen is in liquid form.

The invention discloses novel methods of improving a pre-existing immunity in a mammalian subject using epicutaneous antigen application. The invention may be used to improve, stimulate, reinforce, expand and/or repolarize a pre-existing immune response, which may result from:
- previous conventional vaccination of the subject against a selected pathogen;
- previous conventional priming of the subject against a selected pathogen; and/or
- natural exposure of the subject to a selected pathogen.

In a first embodiment, the invention thus resides in the use of an antigen to stimulate and preferably TH1-polarize an existing immune response against a pathogen in a mammalian subject having received a conventional vaccine, said antigen being administered to the subject by skin application(s). The pre-existing immunity may result from a conventional vaccine performed a long time ago in the subject, e.g., during childhood. The period of time between the conventional vaccine and the skin delivery can indeed vary substantially, as long as the subject still exhibits a pre-existing immunity. The presence of such a pre-existing immunity may be verified, in necessary, by conventional methods. This method is suitable e.g., in the case of subjects vaccinated against hepatitis or tetanus during childhood. The antigen used for skin boost may be inactive or a sub-unit (e.g., influenza, hepatitis B or A, pertussis) or live attenuated (varizella, BCG, polio, influenza, rabies, yellow fever, etc).

In another embodiment, the invention resides in the use of an antigen to stimulate and preferably TH1-polarize an existing immune response against a pathogen in a mammalian subject having received a conventional priming, said antigen being administered to the subject by skin application(s). As illustrated in the examples, the inventors have shown that combining parenteral priming and cutaneous boosting results in a strong and Th1-oriented immune response.

The invention also discloses a method of vaccinating a subject against a pathogen, comprising a first step of conventional priming of the subject with an antigen to cause or stimulate an immune response against said pathogen, followed by a step of boosting the immune response by skin application of an antigen specific for the pathogen.

Priming can comprise either one or several sequential antigen administration(s). In a most typical method, conventional priming comprises one or two antigen administrations. Preferred priming is by injection.

For conventional administration(s), the antigen is typically combined with an adjuvant. Suitable adjuvants include any substance that e.g., activates or accelerates the immune system to cause an enhanced antigen-specific immune response. Examples of adjuvants that can be used in the present invention include mineral salts, such as calcium phosphate, aluminium phosphate and aluminium hydroxide; immunostimulatory DNA or RNA, such as CpG oligonucleotides; proteins, such as antibodies or Toll-like receptor binding proteins; saponins e.g. QS21; cytokines; muramyl dipeptide derivatives; LPS; MPL and derivatives including 3D-MPL; GM-CSF (Granulocyte-macrophage colony-stimulating factor); imiquimod; colloidal particles; complete or incomplete Freund's adjuvant; Ribi's adjuvant or bacterial toxin e.g. cholera toxin or enterotoxin (LT).

For conventional priming, a priming antigen composition is thus used, which comprises the antigen, typically in combination with an adjuvant. Such composition is generally in liquid form, suitable for injection. The composition may further comprise suitable excipients, such as a diluent, carrier, an isotonic solution, water, etc.

As indicated above, the invention combines conventional priming or vaccination with cutaneous boosting. The step of cutaneous boosting typically comprises applying an antigen preparation on the skin of the subject, under conditions allowing a contact with the skin. Application is typically performed under conditions and/or for a period of time sufficient to allow the antigen to penetrate into the stratum corneum of the epidermis and/or to reach immune cells.

Skin application is preferably performed using a device suitable to maintain contact between the antigen preparation and the skin of the subject. Such devices include, without limitation, a patch, a tape, a dressing, a sheet, or any other form known to those skilled in the art. Preferably, the skin device is a patch, even more preferably an occlusive patch. Preferred patch devices do not alter integrity of the skin.

The results presented in the application show that a pronounced Th1-oriented immune response is produced when the skin boost is performed using an occlusive skin patch device.

In the most preferred embodiment, the method of the invention uses a skin patch device as described in international patent applications WO2002/071950 and WO 2007/122226.

Such a device is occlusive and is configured to use an antigen in dry form, the antigen being maintained on the patch through electrostatic and/or Van der Waals forces, with no added adhesive. The preparation and characteristics of such a device (termed Viaskin®) are disclosed in detail in the above quoted applications.

For the performance of the present invention, it is particularly suited to use a device comprising a backing adapted to create with the skin a hermetically closed chamber, this backing having on its skin facing side within the chamber the dry antigen adhered through electrostatic forces and/or Van der Waals forces. Upon application to the skin, moisture increases in the chamber, leading to antigen dissolution and contacting with the skin.

In another preferred embodiment of the invention, the antigen is applied on the skin of the mammalian using an occlusive patch device comprising a support to which the antigen is bound. Preferably, the antigen is bound to the support of the patch through electrostatic or Van der Waals forces, with no added adhesive. In particular embodiments, the support of the patch may be comprised of glass or polymer chosen from the group consisting of cellulose plastics (CA, CP), polyvinyl chloride (PVC), polypropylenes, polystyrenes, polyurethanes, polycarbonates, polyacrylics, polyolefines, polyesters, polyethylenes and ethylene vinyl acrylates (EVA).

In a most preferred embodiment, the skin boost is performed using a dry antigen preparation, which is preferably applied on the skin using an electrostatic skin device. In this regard, the term "dry" designates the fact that the boosting antigen preparation is substantially powdered, e.g., in the form of particles which may be individualized or agglomerated.

Although less preferred, the boosting antigen preparation may be in liquid form and applied using known devices, such as occlusive devices having a reservoir or a perforated membrane.

In another preferred embodiment, the invention combines a liquid, adjuvanted priming antigen preparation and a dry boosting antigen preparation.

Furthermore, in a more preferred embodiment, the boosting antigen preparation is formulated or used with no adjuvant. Indeed, the invention shows that, even in the absence of added adjuvant in the boosting preparation, the method leads to a strong and TH1-polarized immune response.

In another preferred embodiment, the invention thus combines a liquid, adjuvanted priming antigen preparation and a dry, non-adjuvanted, boosting antigen preparation.

The boosting antigen preparation may contain inert excipients or other ingredients, such as penetration enhancers or hydration reagents. It is preferred, however, to use a boosting antigen preparation containing, as the only active substance, one or several antigens, i.e., devoid of further active substances such as adjuvants or penetration enhancers.

In the boosting step, one or more patch devices may be applied, one or more time, directly to intact skin, without any pre-treatment, preferably on a hairless part of the body. Indeed, although encompassed by the present application, it is not required to treat the skin prior to application of the device. Moreover, the present application surprisingly shows that skin pre-treatment substantially influences the type of immune response induced by the epicutaneous boosts. More particularly, when the antigen preparation is non-adjuvanted and applied on intact skin, the pre-existing immune response is strongly amplified and Th1-polarized. This was totally unexpected and is particularly advantageous.

The invention therefore also discloses a method of inducing or enhancing a Th1-polarized antigen-specific immune response in a subject, the method comprising applying, on intact skin of the subject, a dry and non-adjuvanted preparation of said antigen.

The present invention also discloses a composition comprising an injectable preparation of an antigen specific for a selected pathogen, and a dry, non-adjuvanted preparation of an antigen specific for said selected pathogen, for separate, sequential application to a mammalian subject.

This invention also discloses a composition as defined above for use in inducing, stimulating or amplifying a pathogen-specific, Th1-oriented, immune response in a mammalian subject. The composition may be as defined above for boosting an immune response to a selected disease, in a mammalian subject.

This invention also discloses a method of boosting an immune response in a mammalian subject conventionally immunized against a pathogen, the method comprising applying on non pre-treated skin of said subject, and with no added adjuvant, an antigen specific for the pathogen, such application leading to a boost of the immune response.

The term "conventionally immunized" means that a mammal has previously been exposed to the antigen (i.e., primed) by conventional administration thereof. The results presented by the inventors surprisingly show that an epicutaneous boosting with no adjuvant and no skin pre-treatment, leads to an effective amplification and Th1-orientation of the immune response previously caused or stimulated by conventional administration(s).

In the performance of the present invention, the antigen(s) used for priming and boosting may be the same or different, as long as the antigen(s) is (are) specific to the selected pathogen. Typically the antigen(s) used for priming and boosting contain at least one T-cell and/or B-cell epitope in common. Also, the amount of antigen used may be adapted by the skilled person. Priming steps may be performed following published dosages. For boosting according to the present invention, the amount of antigen on each skin device is typically in the range of 0.1 to 10000 µg, preferably 20 to 5000 µg.

The boost immunization of the method of the invention can comprise one or several boosting applications, which may be performed using the same or distinct antigens, preferably the same antigen, at different time intervals, depending on the subject, the antigen, the skin device, the disease, etc.

In a particular embodiment, each antigen boost comprises between 1 and 10 sequential applications of a skin device, typically, between 1 and 5, preferably 1, 2 or 3, over a period of time which may extend from 1 day to several months. Each application may be separated from another by 1 day to several weeks, typically by 1 week to 10 weeks.

Furthermore, a preferred treatment of the invention typically comprises 1, 2 or 3 antigen boosts.

The first antigen boost may be done any time after priming or exposure to the pathogen, as long as antigen-specific immune cells are still present. Preferably, the first boosting is performed between 2 to 10 weeks after priming.

In a particular embodiment, the invention therefore relates to a conventional prime immunization followed, typically 3-6 weeks later, by a single antigen boost immunization.

If required, a second and, optionally, a third antigen boost can be performed. The second and third boosts may be performed within month time intervals, for example between 2-18 months after the previous boost.

In this regard, in a particular embodiment, the invention relates to a conventional prime immunization followed, typically 3-6 weeks later, by a first antigen boost and, typically 1-18 months later, preferably 1-12 months later, for example 3-6 weeks later, by a second antigen boost.

In another particular embodiment, the invention relates to (i) a conventional prime immunization, (ii) typically 3-6 weeks later, a first antigen boost, (iii) typically 1-18 months later, preferably 1-12 months later, for example 3-6 weeks later, a second antigen boost, and (iv) typically 1-18 months later, preferably 1-12 months later, for example 3-6 weeks later, a third and last antigen boost.

The specific dose of antigen as well as the number of boost applications and duration of contact can be adapted by the skilled artisan, depending on the subject, the nature of the antigen preparation, the type of patch device used, etc. For an occlusive device, such as a Viaskin® device, the duration of contact is preferably comprised between 5 to 72 hours. Preferably, for each application, the device is maintained on the skin for a period from 24 to approximately 60 hours, more preferentially approximately 24 hours to 48 hours, typically for 24 hours, 36 hours or 48 hours.

The treatment may be stopped at any time, e.g., once a sufficient amplification and/or Th1-orientation of the immune response has been established. In this regard, the present invention shows that a specific immune reaction provoked by parenteral priming, which is Th2-oriented, can be efficiently amplified and Th1-polarized upon epicutaneous boosting. Th1 and Th2 cells are two types of CD4+ helper T-cells which differ in their pattern of cytokines production. Th1 cells produce IFN-γ, IL-2 and TNF-β and are involved in cell-mediated immune responses that are beneficial in host-defence against intracellular pathogens and malignant cells. Th2 cells secrete IL-4, IL-5, IL-9, IL-10 and IL-13, which increase antibody responses, including IgE production. Th1 and Th2 responses are mutually antagonistic, such that they normally exist in equilibrium and cross-regulate each other.

Within the context of this invention, a Th1 orientation of the immune response means that the ratio of Th1/Th2 immune cells or cytokines is increased, preferably by at least 10%, as a result of the boosting of the present invention.

As disclosed in the experimental section, a supernatant of T-cells, extracted from subjects boosted according to the invention, contains significantly higher concentrations of IFN-γ (which is the main Th1 cytokine) and significantly lower concentrations of IL-5, in comparison with subjects boosted by conventional route of administration, thus demonstrating a Th1 orientation of the immune response. Th1 orientation indicates, in a specific embodiment, that the concentration of IFN-γ, or the number of IFN-γ -producing cells, increases by at least 10% as compared to subjects treated using conventional route of administration, and/or that the concentration of IL-5, or the number of IL-5-producing cells, decreases by at least 10% as compared to subjects treated using conventional route of administration.

This invention also discloses a method of amplifying and Th1 orienting an immune response against a pathogen in a conventionally immunized mammalian subject, the method comprising applying on the skin of said parenterally immunized subject an antigen specific for the pathogen, with no added adjuvant.

The invention also discloses a method of amplifying and Th1 orienting an immune response against a pathogen in a conventionally immunized mammalian subject, the method comprising applying on non pre-treated skin of said parenterally immunized subject an antigen specific for the pathogen, with no added adjuvant.

The following examples are given for purposes of illustration and not by way of limitation.

### EXAMPLES

### Example 1: Amplification and TH1-orientation of the immune response by skin boosting

### A. Material & Methods

### Animals

Adult BALB/c mice were purchased from Charles River Laboratories (France). All experiments were performed according to European Community rules of animal care.

### Antigen

### Surface antigen of Hepatitis B virus, i.e., HBsAg

### Skin Device

Viaskin® device as described in WO 2007/122226. The device is occlusive and electrostatic. The antigen is maintained on the surface of the backing support, in dry and non-adjuvanted form, through electrostatic forces.

### Quantification of specific IgE, IgG1, IgG2a

Blood samples were collected from retro-orbital venous plexus before and during immunotherapy and the plasma were stored at -30°C until further analyses.

A qualitative ELISA, validated using ICH guidelines, was used for specific IgG1 and IgG2a. Briefly, microtiter plates were coated with HBsAg at a concentration of 2 µg/ml. Serial dilutions of 100 µl of each serum were dispensed per well and incubated for 24 h at 4°C. An anti-mouse IgG1 or IgG2a antibody labelled with peroxydase was used as a tracer. Reagent (TMB) was used as an enzyme substrate. Dilution curves were plotted and the titration of the serum was determined at the same ordinate (y axis).

### Cytokine production

After the last blood sampling, mice were killed by vertebral dislocation and spleens were harvested under sterile conditions.

### ELISA quantification

Cell culture were performed in the presence of HBsAg (0 - 50 µg.ml⁻¹) in 24-wells microtitre plates (2.10⁶ cells/well). After 72hrs in vitro stimulation, supernatants were harvested and the quantification of IL-5 and IFNγ were assayed using CytoSetTM kits (BioSource International Europe, Belgium) according to the manufacturer's instructions.

### ELISpot

Cell culture were performed in the presence of HBsAg (0 - 50 µg.ml⁻¹), in ELISpot microtitre plates (R&D system, USA) according to the recommendation of the manufacturer (BD, USA). The quantification of IL-5 and IFNγ were determined after 48hrs of intro stimulation.

### Protocol

- **Priming :** adult BALB/C mice (2 groups of 7) were immunized conventionally (i.m. injection) with 2mg HBsAg/10µg AlOH
- **Day 28 post-priming**
   - Serum collected
   - Group 1 "HBsAg/Alum": boost with 2mg HBsAg/10µg AlOH
   - Group 2 "Viaskin®/HBsAg patches": boost with Viaskin® loaded with 100mg HBsAg. The Viaskin® was removed 48 hours after application.
- **Day 49 post-priming** (21 days after post-boost I)
   - Serum collected
   - Group 1: no boost
   - Group 2: boost with Viaskin® loaded with 100mg HBsAg

The patches for boost 1 and boost 2 were prepared with 2 different methods: For boost 1, patches were prepared by lyophilisation and for boost 2, patches were prepared by drying. In the two preparations, however, the antigen is loaded as powder on the patches.
- **Day 63 post-priming** (14 days after post-boost II)
   - Serum collected
      Spleens were taken for analysis of T cell responses
- **Antibody quantification :** serum anti-HBsAg antibodies (IgGl and IgG2a) were quantified by ELISA using antigen-coated plates on day 0, 28, 49 and 63.
- **T cell responses :**

### Ex vivo :

- Splenocytes were cultured with or without HBsAg (5mg/ml) for 1 hour before addition of Brefeldin A/monensin, to block cytokine secretion.
- After 6 additional hours of culture, cells were stained with antibodies against surface molecules, then fixed and permeabilized prior to staining with antibodies against IL-5, IL-2, IFN-γ.
- Results (not shown) : cytokine-producing cells were not detected above background in any experimental group (*except in mitogen-stimulated control splenocytes)*

### After in vitro restimulation:

- Splenocytes were cultured with or without antigen (5mg/ml & 20mg/ml) prior to analyses for 72 hours.
   - IL-5 and IFN-g producing splenocytes were quantified by ELISpot after 48 hours of culture with or without HBsAg .
   - Cytokine secretion was determined in culture supernatants by ELISA after 72h of culture with or without HBsAg .

### Statistical analysis

The Graph Pad Software (San Diego, USA) was used for statistical analysis. Data were analysed using analysis of variance (ANOVA) and Dunnett's test when comparing treated mice with controls, or using ANOVA and Tukey's test when comparing all the groups with each other.

### B. Results

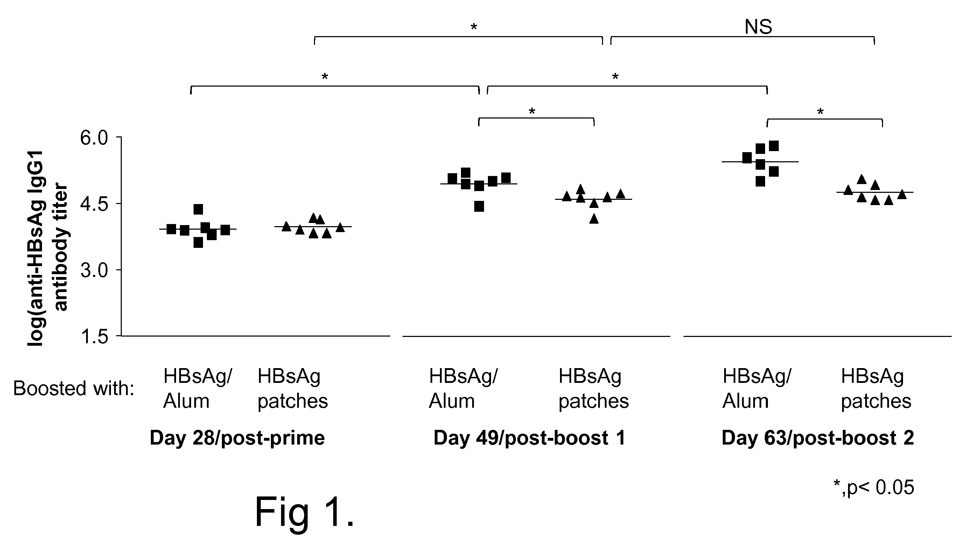

### Influence of epicutaneous boost of the invention on anti-HBsAg IGg1 responses (fig 1)

The results presented show a significant boosting of IgG1 by the first skin patch application (*see* *Fig. 1**, Comparison of D49 and D28.*

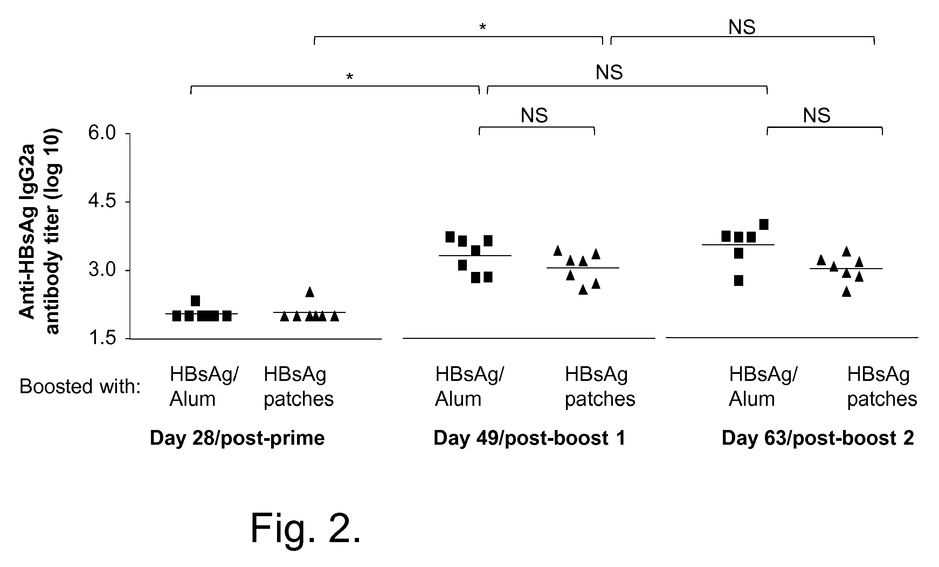

### Influence of epicutaneous boost of the invention on anti-HBsAg IgG2a responses (fig 2)

The results presented show a significant boosting of IgG2a by the first skin patch application (*see* *Fig. 1**, Comparison of D49 and D28.*

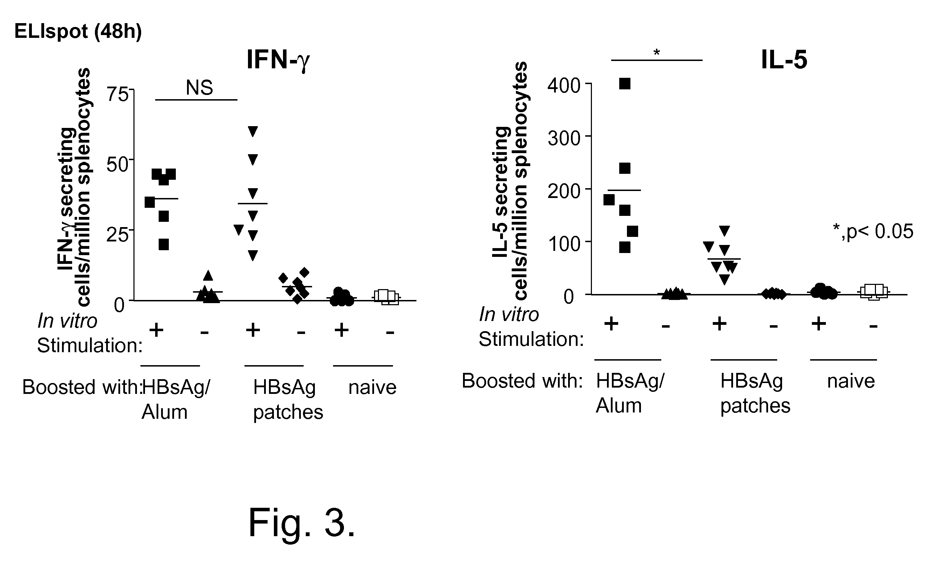

### Cytokine-producing-cells in mice boosted twice with epicutaneous boost of the invention or HBsAg/AlOH (ELISpot) (fig 3)

The results presented Fig. 3 show that the number of IFN-γ-producing cells is similar in mice boosted with Viaskin® or intra-muscular injection with adjuvant, whereas IL-5 producing cells are fewer in mice boosted with Viaskin®. Thus, despite a Th2-inducing priming with HBsAg/AlOH, Viaskin® boosting elicited balanced Th1/Th2 responses, contrasting with the preferential Th2 response of HBsAg/AlOH (by intra-muscular injection).

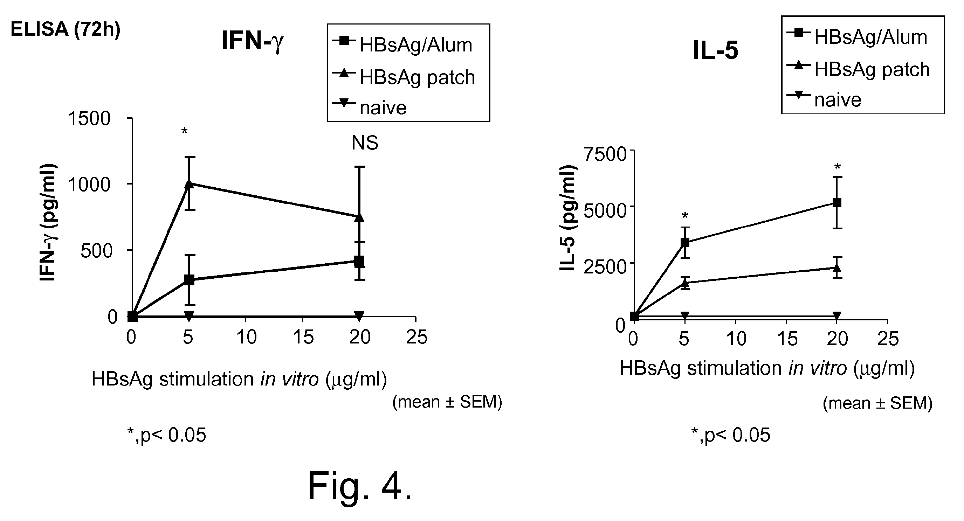

### Cytokine secretion in mice boosted twice with epicutaneous boost of the invention or HBsAg/AlOH (ELISA) (fig 4)

At 72h, IFN-γ is present at significantly higher concentrations and IL-5 at significantly lower concentrations in the supernatant of T cells from mice boosted with Viaskin than with HBsAg/AlOH (by intra-muscular injection).

### IL-5 responses in mice boosted twice with epicutaneous boost of the invention or HBsAg/AlOH (ELISA - ELISpot details)

- At 72h, IL-5 is present at higher concentrations in mice boosted with Viaskin® than in naive mice.
- At 72h, IL-5 is present at significantly lower concentrations in mice boosted with Viaskin® than with HBsAg/AlOH.
- The same pattern is observed regardless of the restimulation conditions (in vitro with ELISA quantification of in vitro by ELISpot).

### IFN-g responses in mice boosted twice with epicutaneous boost of the invention or HBsAg/AlOH (ELISA, ELISpot details)

- Splenocytes from immune mice spontaneously release IFN-g, and this release was higher in mice boosted with Viaskin®.
- If this spontaneous release is subtracted :
   - IFN-γ is not detected at significantly higher concentrations in mice boosted with HBsAg/AlOH than in naïve mice.
   - IFN-γ is detected at significantly higher concentrations in mice boosted with Viaskin® than in naive mice.

### C. Conclusions

The skin boost of the invention significantly increased anti-HBsAg IgG1 and IgG2a responses in mice conventionally primed with HBsAg/AlOH. IgG1 and IgG2a levels were similar to those obtained by conventional boost (intramuscular injection with adjuvant). The skin boosting increased IFN-γ and reduced IL-5 anti-HBsAg responses.

These results demonstrate, in vivo, a successful amplification and Th1 re-polarization of the Th2-type responses elicited by conventional HBsAg/ALOH priming.

### Example 2: Effect of skin pre-treatment on the type of immune response

### A. Materials and Methods

BALB/c mice were intragastrically sensitized to peanut protein extract (PPE) mixed to cholera toxin as adjuvant for 6 weeks (once a week).

Then, an immunotherapy was conducted for 8 consecutive weeks. 10 sensitized mice were epicutaneously treated on intact skin. 10 sensitized mice were epicutaneously treated on skin pre-treated by 10 tape-stripping. Tape-stripping comprised 10 stripping with an adhesive, which led to a substantial alteration of the stratum corneum integrity. 10 sensitized mice were not treated. 10 naive mice served as controls.

At the end of immunotherapy, all the mice were exposed to a 10-day sustained oral peanut regimen. After sacrifice, the lesions were evaluated in the esophagus and jejunum by histological analysis. The expression of mRNA of Th1, Th2 and Treg cytokines were measured by TR-qPCR.

The measurements of serological responses (specific IgE, IgG1 and IgG2a) were done by ELISA, before and at the end of immunotherapy.

### B. Results

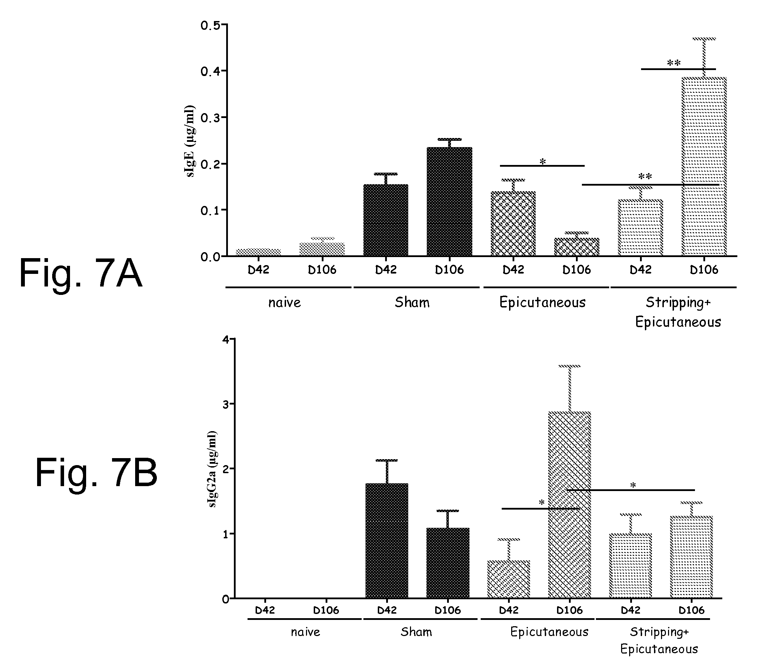

Following epicutaneous treatment on intact skin, sIgE decreased and sIgG2a increased, respectively 0.036±0.01 vs 0.231±0.02µg/ml (EPIT vs Sham, p<0.05) and 2.86±0.78 vs 1,06±0.28µg/ml (p<0.05). In contrast, following immunotherapy on tape-stripped-skin, sIgE increased and sIgG2a was not modified, respectively 0.383±0.08µg/ml (p<0.01 vs Sham) and 1.252±0.22µg/ml (see Figures 7A and 7B).

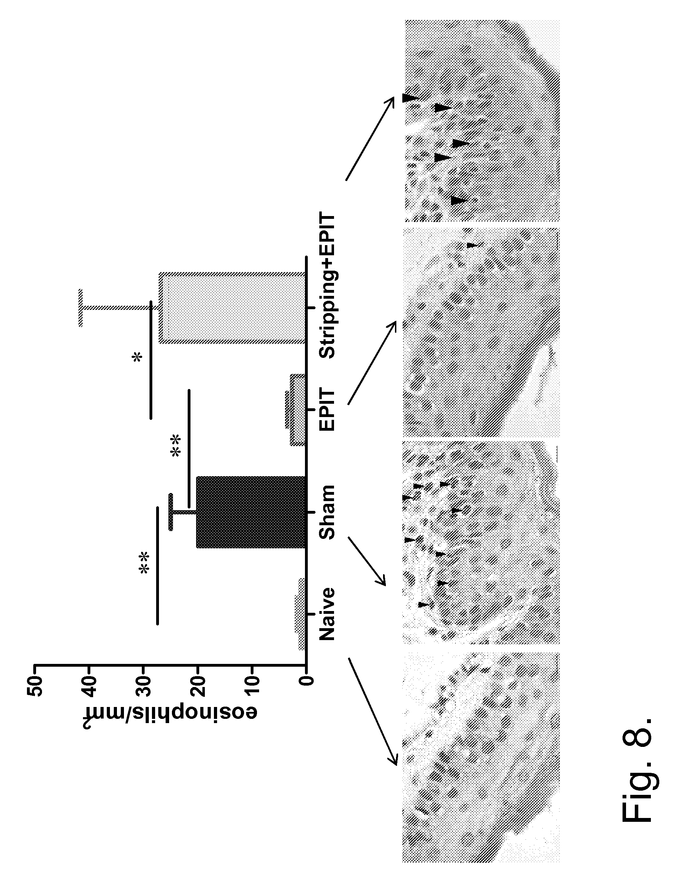

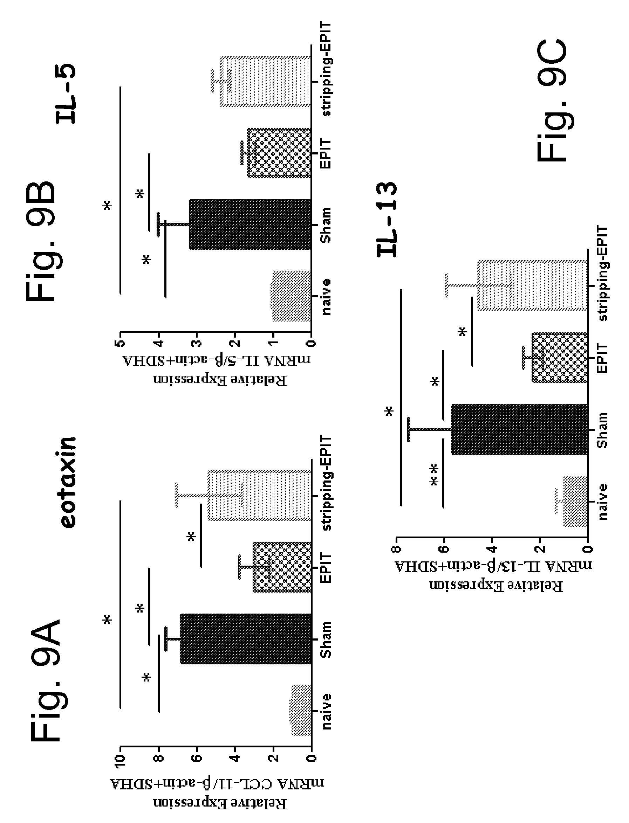

The results also show that eosinophilic concentration in oesophageal mucosa was substantially higher in Sham and stripped skin treated mice, 19.9±1.5 and 26.7±8.7, than in mice treated on intact skin, 2.7±0.9 (p<0.01 and p<0.05) and Naive, 1.1±0.7cells/mm2 (p<0.01) (see figure 8). Furthermore, eotaxin, IL-5 and IL-13 mRNA expressions were significantly reduced by epicutaneous treatment on intact skin (respectively 1.14, 1.35, 1.02) as compared to Sham (2.57, 2.60, 2.50, p<0.05) and stripped-skin treatment (2.02, 1.95, 2.01, p<0.05) (see Figures 9A, B and C).

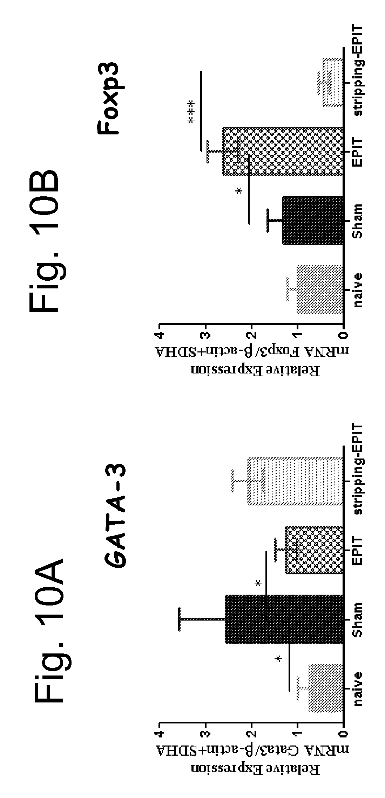

Interestingly, GATA-3 mRNA, which is a Th2 transcription factor, was significantly decreased in mice treated on intact skin compared to Sham group (p<0.05), as shown in Figure 10A. Also, Foxp3, a Treg transcription factor, was significantly increased in mice tread on intact skin as compared to Sham (p>0.05) and Stripped-skin (p<0.001) (see Figure 10B).

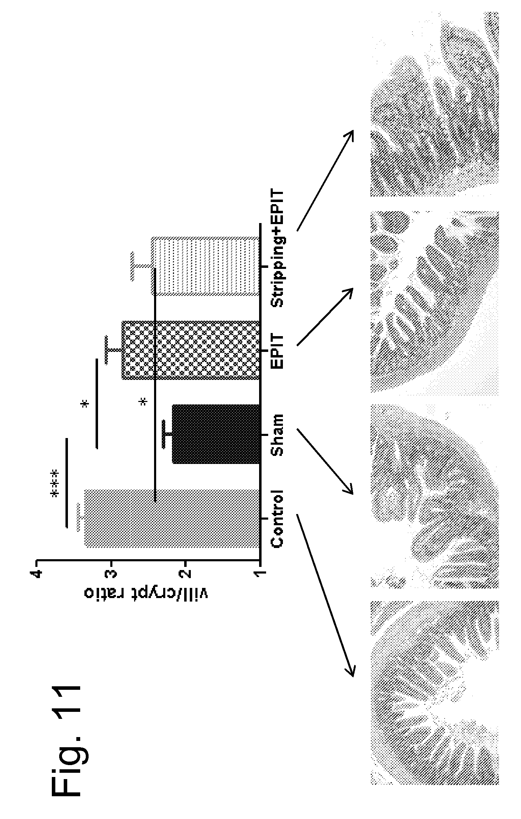

Finally, as shown in Figure 11, the duodenal villus/crypt-ratio was significantly lower in Sham (2.1±0.2) and stripped-skin treated mice (2.4±0.3) than in intact skin treated (2.9±0.2) (p<0.05) and Naive (3.3±0.1) mice (p<0.001 and p<0.05).

### C. Discussion

The results obtained show that in epicutaneous immunotherapy, the profile of the immunological response provoked depends on the level of integrity of the stratum corneum:
On intact skin, a decrease of IgE / increase of IgG2a is induced
On treated skin with altered stratum corneum : an increase of IgE / no IgG2a is induced.

Also, after a peanut exclusive diet: high infiltration of eosinophils was observed only for mice treated on stripped skin.

### Conclusions

The results show that an efficient immune response can be obtained and amplified by epicutaneous therapy using non-adjuvanted antigen boosts applied using an occlusive device. The boost causes a Th1 oriented and particularly strong immune response when the antigen is applied on intact skin.

### REFERENCES

Glenn, Scharton-Kersten et al. 1999, "Advances in vaccine delivery: transcutaneous immunisation." Expert Opin Investig Drugs 8(6): 797-805;
Kaiserlian and Etchart 1999 "Epicutaneous and transcutaneous immunization using DNA or proteins." Eur J Dermatol 9(3): 169-76;
Partidos, Beignon et al. 2003, "Delivering vaccines into the skin without needles and syringes." Expert Rev Vaccines 2(6): 753-61 ; "Immunity under the skin: potential application for topical delivery of vaccines." Vaccine 21(7-8): 776-80).
Shiver, JW et al., 2002, Replication-incompetent adenoviral vaccine vector elicits effective anti-immunodeficiency-virus immunity." Nature 415: 331-335.
Woodland DL et al., 2004, "Jump-starting the immune system: prime-boosting comes of age." Trends Immunol. 25: 98-104.
Wu, L et al., 2005, "Enhanced breadth of CD4 T-cell immunity by DNA prime and adenovirus boost immunization to human immunodeficiency virus Env and Gag immunogens." J Virol 79: 8024-8031.
McConnell et al., 2007, "Adenovirus-based prime-boost immunization for rapid Vaccination against Anthrax. » Molecular Therapy 15: 203-210.

## Claims

1. A preparation of an antigen specific for an infectious pathogen for use in amplifying a pre-existing immune response against said pathogen in a mammalian subject, said pre-existing immune response resulting from the prior parenteral immune priming or vaccination of the subject against said pathogen, wherein said antigen preparation is non-adjuvanted and applied to intact skin of the subject with a skin patch device, and wherein said amplified immune response is Th1-oriented.

2. The antigen preparation for use in amplifying a pre-existing immune response of claim 1, wherein said antigen preparation is dry.

3. The antigen preparation for use in amplifying a pre-existing immune response of claim 1, wherein said antigen preparation is applied to the skin of the subject after cleaning of the skin surface at the site of application to remove corneocytes.

4. The antigen preparation for use in amplifying a pre-existing immune response of any one of claims 1 to 2, wherein said antigen preparation is applied on a non-pretreated area of the skin having a substantially non altered stratum corneum.

5. The antigen preparation for use in amplifying a pre-existing immune response of anyone of claims 1 to 4, wherein said antigen preparation is applied on the skin of the subject using an occlusive patch device.

6. The antigen preparation for use in amplifying a pre-existing immune response of claim 5, wherein the occlusive patch device comprises a support to which the antigen is bound through electrostatic or Van der Waals forces with no added adhesive.

7. The antigen preparation for use in amplifying a pre-existing immune response of anyone of claims 1 to 6, wherein said antigen is administered as 1, 2 or 3 antigen skin boosts to the subject.

8. The antigen preparation for use in amplifying a pre-existing immune response of claim 7, which comprises a first antigen skin boost 2-8 weeks after priming, optionally a second antigen skin boost 1-12 months after the first boost and, optionally, a third antigen skin boost 1-12 months after the second boost.

9. The antigen preparation for use in amplifying a pre-existing immune response of any of the preceding claims, wherein the same antigen is used for skin boost and prior priming or vaccination.

10. The antigen preparation for use in amplifying a pre-existing immune response of any one of claims 1 to 9, wherein the infectious pathogen is a virus, a bacterium, a parasite, or a fungus.

11. The antigen preparation for use in amplifying a pre-existing immune response of any of the preceding claims, wherein the antigen is a virus or bacterial surface antigen.

12. A non-adjuvanted antigen preparation specific for an infectious pathogen for use in boosting an immune response in a mammalian subject pre-immunized against said pathogen by parenteral priming or vaccination, the antigen preparation being applied in dry form on intact skin of said subject, such application causing an amplification and a Th1 orientation of the immune response by increasing by at least 10% the ratio of Th1/Th2 immune cells or cytokines.

13. A non-adjuvanted antigen preparation specific for an infectious pathogen for use in a method for inducing or stimulating a pathogen-specific, Th1-oriented immune response in a mammalian subject, the method comprising:
- parenterally administering to the mammalian an antigen specific for the pathogen to cause or stimulate an immune response against said pathogen, and
- subsequently applying on intact skin of the mammalian an antigen specific for the pathogen, allowing amplification of a Th1-oriented immune response.

## Patentansprüche

1. Eine Zubereitung eines für einen infektiösen Erreger spezifisches Antigens zur Verwendung in der Amplifizierung einer bereits bestehenden Immunantwort gegen den Erreger in einem Säugersubjekt, wobei die bereits bestehende Immunantwort aus dem vorausgehenden parenteralen Immunprimen oder der Impfung des Subjekts gegen den Erreger resultiert, wobei die Antigenzubereitung nicht-adjuvant ist und auf intakter Haut des Subjekts mit einer Haut-Pflaster-Vorrichtung appliziert wird und wobei die amplifizierte Immunantwort Th1-orientiert ist.

2. Die Antigenzubereitung zur Verwendung in der Amplifizierung einer bereits bestehenden Immunantwort nach Anspruch 1, wobei die Antigenzubereitung trocken ist.

3. Die Antigenzubereitung zur Verwendung in der Amplifizierung einer bereits bestehenden Immunantwort nach Anspruch 1, wobei die Antigenzubereitung auf die Haut des Subjekts nach dem Reinigen der Hautoberfläche am Applikationsort, um Korneozyten zu entfernen, appliziert wird.

4. Die Antigenzubereitung zur Verwendung in der Amplifizierung einer bereits bestehenden Immunantwort nach einem der Ansprüche 1 bis 2, wobei die Antigenzubereitung auf einen nicht vorbehandelten Bereich der Haut, die ein im Wesentlichen nicht verändertes Stratum corneum hat, appliziert wird.

5. Die Antigenzubereitung zur Verwendung in der Amplifizierung einer bereits bestehenden Immunantwort nach einem der Ansprüche 1 bis 4, wobei die Antigenzubereitung auf die Haut des Subjekts mittels einer Okklusivpflaster-Vorrichtung appliziert wird.

6. Die Antigenzubereitung zur Verwendung in der Amplifizierung einer bereits bestehenden Immunantwort nach Anspruch 5, wobei die Okklusivpflaster-Vorrichtung einen Träger umfasst, an den das Antigen durch elektrostatische oder Van der Waals Kräfte ohne hinzugefügten Klebstoff gebunden ist.

7. Die Antigenzubereitung zur Verwendung in der Amplifizierung einer bereits bestehenden Immunantwort nach einem der Ansprüche 1 bis 6, wobei das Antigen als 1, 2 oder 3 Antigen Hautankurbelungen an das Subjekt verabreicht wird.

8. Die Antigenzubereitung zur Verwendung in der Amplifizierung einer bereits bestehenden Immunantwort nach Anspruch 7, die eine erste Antigen-Hautankurbelung 2 bis 8 Wochen nach dem Primen, optional eine zweite Antigen-Hautankurbelung 1 bis 12 Monate nach der ersten Ankurbelung und optional eine dritte Antigen-Hautankurbelung 1 bis 12 Monate nach der zweiten Ankurbelung umfasst.

9. Die Antigenzubereitung zur Verwendung in der Amplifizierung einer bereits bestehenden Immunantwort nach einem der vorangegangenen Ansprüche, wobei das gleiche Antigen zur Hautankurbelung und vorherigem Primen oder Impfung verwendet wird.

10. Die Antigenzubereitung zur Verwendung in der Amplifizierung einer bereits bestehenden Immunantwort nach einem der Ansprüche 1 bis 9, wobei der infektiöse Erreger ein Virus, ein Bakterium, ein Parasit oder ein Pilz ist.

11. Die Antigenzubereitung zur Verwendung in der Amplifizierung einer bereits bestehenden Immunantwort nach einem der vorangegangenen Ansprüche, wobei das Antigen ein Virus oder ein bakterielles Oberflächenantigen ist.

12. Eine nicht-adjuvante Antigenzubereitung spezifisch für einen infektiösen Erreger zur Verwendung in der Ankurbelung einer Immunantwort in einem Säugersubjekt, das preimmunisiert ist gegen den Erreger durch parentales Primen oder Impfung, wobei die Antigenzubereitung in trockener Form auf intakter Haut des Subjekts appliziert wird, solch Applikation verursacht eine Amplifizierung und eine Th1 Orientierung der Immunantwort durch Steigerung des Verhältnisses von Th1/Th2 Immunzellen oder Zytokinen um mindestens 10%.

13. Eine nicht-adjuvante Antigenzubereitung spezifisch für einen infektiösen Erreger zur Verwendung in einem Verfahren zur Induktion oder Stimulation einer Erregerspezifischen, Th1-orientierten Immunantwort in einem Säugersubjekt, das Verfahren umfassend:
- parenterales Verabreichen eines für den Erreger spezifisches Antigens an den Säuger um eine Immunantwort gegen den Erreger auszulösen oder zu stimulieren und
- anschließendes Applizieren eines für den Erreger spezifisches Antigens auf intakte Haut des Säugers, was die Amplifizierung einer Th1-orientierten Immunantwort erlaubt.

## Revendications

1. Préparation d'un antigène spécifique d'un pathogène infectieux pour son utilisation pour amplifier une réponse immune préexistante contre ledit agent pathogène chez un sujet mammifère, ladite réponse immune préexistante résultant d'une activation immune (« priming ») ou d'une vaccination parentérale antérieure du sujet contre ledit agent pathogène, ladite préparation d'antigène étant sans adjuvant et appliquée sur la peau intacte du sujet avec un dispositif patch cutané, et ladite réponse immune amplifiée étant orientée Th1.

2. Préparation d'antigène pour une utilisation pour amplifier une réponse immune préexistante selon la revendication 1, ladite préparation d'antigène étant sèche.

3. Préparation d'antigène pour une utilisation pour amplifier une réponse immune préexistante selon la revendication 1, ladite préparation d'antigène étant appliquée sur la peau du sujet après nettoyage de la surface de peau au niveau du site de l'application pour enlever des corneocytes.

4. Préparation d'antigène pour une utilisation pour amplifier une réponse immune préexistante selon l'une quelconque des revendications 1 à 2, ladite préparation d'antigène étant appliquée sur une partie non prétraitée de la peau ayant une couche cornée substantiellement non altérée.

5. Préparation d'antigène pour une utilisation pour amplifier une réponse immune préexistante selon l'une quelconque des revendications 1 à 4, ladite préparation d'antigène étant appliquée sur la peau du sujet au moyen d'un dispositif patch occlusif.

6. Préparation d'antigène pour une utilisation pour amplifier une réponse immune préexistante selon la revendication 5, ledit dispositif patch occlusif comprenant un support auquel l'antigène est lié par des forces électrostatiques ou de Van der Waals sans ajout d'adhésif.

7. Préparation d'antigène pour une utilisation pour amplifier une réponse immune préexistante selon l'une quelconque des revendications 1 à 6, ledit antigène étant administré en tant que 1, 2 ou 3 rappels antigéniques (« boosts ») cutanés au sujet.

8. Préparation d'antigène pour une utilisation pour amplifier une réponse immune préexistante selon la revendication 7, comprenant un premier rappel antigénique (« boost ») cutané 2-8 semaines après le priming, optionnellement un deuxième rappel antigénique (« boost ») cutané 1-12 mois après le premier rappel (« boost ») et, optionnellement, un troisième rappel antigénique (« boost ») cutané 1-12 mois après le deuxième rappel (« boost »).

9. Préparation d'antigène pour une utilisation pour amplifier une réponse immune préexistante selon l'une quelconque des revendications précédentes, où le même antigène est utilisé pour le rappel (« boost ») cutané et pour l'activation (« priming ») ou la vaccination antérieure.

10. Préparation d'antigène pour une utilisation pour amplifier une réponse immune préexistante selon l'une quelconque des revendications 1 à 9, où l'agent pathogène infectieux est un virus, une bactérie, un parasite, ou un champignon.

11. Préparation d'antigène pour une utilisation pour amplifier une réponse immune préexistante selon l'une quelconque des revendications précédentes, où l'antigène est un antigène de surface viral ou bactérien.

12. Préparation non adjuvantée d'antigène spécifique d'un pathogène infectieux pour une utilisation pour amplifier une réponse immune chez un sujet mammifère pré-immunisé contre ledit pathogène par une activation (« priming ») ou une vaccination parentérale, la préparation d'antigène étant appliquée sous forme sèche sur la peau intacte dudit sujet, ladite application entraînant une amplification et une orientation Th1 de la réponse immune en augmentant d'au moins 10% le rapport Th1/Th2 des cellules immunes ou des cytokines.

13. Préparation non adjuvantée d'antigène spécifique d'un pathogène infectieux pour une utilisation dans une méthode pour induire ou stimuler une réponse immune spécifique du pathogène, orientée Th1, chez un sujet mammifère, la méthode comprenant :
- l'administration parentérale au mammifère d'un antigène spécifique du pathogène pour provoquer ou stimuler une réponse immune contre ledit pathogène, et
- ensuite, l'application sur peau intacte du mammifère d'un antigène spécifique du pathogène, permettant l'amplification d'une réponse immune orientée Th1.
